# EUROPEAN PATENT APPLICATION

(11) **EP 4 059 543 A1**
(43) Date of publication of application: **21.09.2022**
(21) Application number: 20887578.1
(22) Date of filing: 13.11.2020
(51) Int. Cl.: A61M 5/158, A61M 25/06

(54) **INDWELLING NEEDLE**

(30) Priority: 14.11.2019 JP 2019206240
(71) Applicant: Nipro Corporation, Osaka-shi, Osaka 531-8510 (JP)
(72) Inventor: SAKAMOTO, Shingo, Osaka-shi, Osaka 531-8510 (JP)
(74) Representative: Novagraaf International SA
(86) International application number: PCT/JP2020/042504
(87) International publication number: WO 2021/095873

(57) **Abstract**

The present invention is an indwelling needle characterized by comprising: an inner needle that punctures a body; an outer needle that is inserted into the body together with the inner needle as the inner needle penetrates the outer needle and punctures the body; a hub that has a passage in communication with the interior of the outer needle once the base end of the outer needle is secured and the inner needle is removed; and an elastic member that is disposed within the hub, is penetrated by the inner needle, and blocks the base end side of the hub once the inner needle is removed, wherein a port through which a fluid flows to the passage is provided to a side surface of the hub, the passage includes a switching part that switches the flow direction of the fluid that has flowed in from the axial direction of the outer needle to the axial direction of the port or switches the flow direction of the fluid that has flowed in from the axial direction of the port to the axial direction of the outer needle, and the passage in the switching part is at least partially formed from the elastic member and has a circular cross section.

## Description

### Technical Field

The present invention relates to an indwelling needle including an outer needle that is made to puncture a living body and left indwelling in the living body.

### Background

As medical instruments used in a case of performing artificial dialysis, an indwelling needle, a catheter tube (for example, Patent Literature 1), and the like have been developed. An indwelling needle includes an inner needle that is made to puncture a blood vessel of a patient for whom to perform artificial dialysis, and an outer needle into which the inner needle penetrates and which is inserted into a blood vessel of the patient together with the inner needle when the inner needle is made to puncture a living body. Then, the outer needle is left indwelling in the blood vessel of the patient, and blood is collected from the patient via the outer needle, or dialyzed blood is returned to the patient's body.

### Citation List

### Patent Literature

Patent Literature 1: JP 2009-534090 A

### Summary of Invention

### Technical Problem

It is conceivable that a main body side surface of the indwelling needle as described above is provided with a port that communicates with a hollow portion of the outer needle and allows blood supplied from an external device such as an artificial dialysis device to flow into the hollow portion of the outer needle as in Patent Literature 1. However, in a case where there is a flow path (for example, a flow path between the port and seal rubber in Patent Literature 1) deviated from flow of blood formed by an inlet and an outlet in an indwelling needle main body, blood stagnates in the flow path. As a result, it is conceivable that inconveniences such as coagulation of blood in the flow path and return of a solidified clot to the patient's body occur.

In view of the above, an object of the present application is to provide a technique for suppressing stagnation of blood inside an indwelling needle.

### Solution to Problem

In order to solve the above-described problem, the present invention employs a configuration below.

That is, an indwelling needle according to an aspect of the present invention is an indwelling needle including an inner needle that punctures a living body, an outer needle through which the inner needle penetrates, the outer needle being inserted into the living body together with the inner needle as the inner needle punctures the living body, a hub having a flow path communicating with an inside of the outer needle in a case where a base end of the outer needle is fixed and the inner needle is removed, and an elastic member that is arranged inside the hub and through which the inner needle penetrates, the elastic member closing a base end side of the hub in a case where the inner needle is removed. A port through which fluid flows to the flow path is provided on a side surface of the hub, the flow path includes a conversion portion in which a flow direction of fluid flowing in from an axial direction of the outer needle is converted to an axial direction of the port or a flow direction of fluid flowing in from the axial direction of the port is converted to the axial direction of the outer needle, and at least a part of the flow path in the conversion portion is formed of the elastic member, and has a cross section of a circular shape.

According to this configuration, when a flow direction of blood is converted to a direction from a blood vessel of a patient to the port, since a portion around an axis with respect to the flow direction is smooth, the blood smoothly flows in the flow direction, and formation of a stagnation portion of blood in a part of the flow path in the conversion portion is suppressed.

Further, conversely, when blood to be supplied to a blood vessel of a patient flows from the port toward the inside of the outer needle, stagnation of the blood in the conversion portion is also suppressed. Further, the circular shape here does not mean only a perfect circle, and includes a shape close to a circle. More specifically, it is a concept including not only an elliptical shape, an oval shape, and an egg shape but also a substantially circular shape with a slight variation in radius.

Further, an indwelling needle according to an aspect of the present invention may be an indwelling needle including an inner needle that punctures a living body, an outer needle through which the inner needle penetrates, the outer needle being inserted into the living body together with the inner needle as the inner needle punctures the living body, a hub having a flow path communicating with an inside of the outer needle in a case where a base end of the outer needle is fixed and the inner needle is removed, and an elastic member that is arranged inside the hub and through which the inner needle penetrates, the elastic member closing a base end side of the hub in a case where the inner needle is removed. A port through which fluid flows to the flow path is provided on a side surface of the hub, the flow path has a conversion portion at least a part of which is formed of the elastic member and in which a flow direction of fluid flowing in from an axial direction of the outer needle is converted to an axial direction of the port, at least a part of a connection portion between the flow path and the port is formed of the elastic member, and a cross-sectional shape of an opening portion of the port with respect to the flow path in the connection portion is the same as a cross-sectional shape of an opening portion of the flow path with respect to the port.

According to the configuration, the cross-sectional shape of the opening portion with respect to the port of the flow path can be easily matched with the cross-sectional shape of the opening portion with respect to the flow path of the port by the elastic member. As a result, in the connection portion, the side wall forming the flow path and the side wall inside the port can be made smoothly continuous. Therefore, stagnation of blood at the connection portion is suppressed. Note that the opening portion with respect to the port of the flow path may be formed of the elastic member and the hub or another member inserted into the hub, or may be formed of the elastic member alone.

Further, an indwelling needle according to an aspect of the present invention may be an indwelling needle including an inner needle that punctures a living body, an outer needle through which the inner needle penetrates, the outer needle being inserted into the living body together with the inner needle as the inner needle punctures the living body, a hub having a flow path communicating with an inside of the outer needle in a case where a base end of the outer needle is fixed and the inner needle is removed, and an elastic member that is arranged inside the hub and through which the inner needle penetrates, the elastic member closing a base end side of the hub in a case where the inner needle is removed and connecting with the base end of the outer needle at a front end. A port through which fluid flows to the flow path is provided on a side surface of the hub, the flow path has a conversion portion at least a part of which is formed of the elastic member and in which a flow direction of fluid flowing in from an axial direction of the outer needle is converted to an axial direction of the port, and the front end of the elastic member extends further on a front end side than the port.

According to the configuration, the positions of the outer needle base end and an elastic valve element are close, a space between the outer needle base end and the elastic valve element can be reduced, and stagnation of fluid is suppressed.

Further, an indwelling needle according to an aspect of the present invention may be an indwelling needle including an inner needle that punctures a living body, an outer needle through which the inner needle penetrates, the outer needle being inserted into the living body together with the inner needle as the inner needle punctures the living body, a hub having a flow path communicating with an inside of the outer needle in a case where a base end of the outer needle is fixed and the inner needle is removed, and an elastic member that is arranged inside the hub and through which the inner needle penetrates, the elastic member closing a base end side of the hub in a case where the inner needle is removed. A port through which fluid flows to the flow path is provided on a side surface of the hub, and at least a part of the flow path is a tunnel-shaped flow path formed in the elastic member.

According to the configuration, at least a part of the flow path is a tunnel-shaped flow path formed in the elastic member, and a side wall of the flow path is formed only from a surface of the elastic member. Therefore, it is possible to reduce a space in which blood may stagnate in the flow path between the outer needle and the port, and fluid can smoothly flow in the flow path.

Further, an indwelling needle according to an aspect of the present invention may be an indwelling needle including an inner needle that punctures a living body, an outer needle through which the inner needle penetrates, the outer needle being inserted into the living body together with the inner needle as the inner needle punctures the living body, a hub having a flow path communicating with an inside of the outer needle in a case where a base end of the outer needle is fixed and the inner needle is removed, and an elastic member that is arranged inside the hub and through which the inner needle penetrates, the elastic member closing a base end side of the hub in a case where the inner needle is removed. A port through which fluid flows to the flow path is provided on a side surface of the hub, the flow path has a conversion portion at least a part of which is formed of the elastic member and in which a flow direction of fluid flowing in from an axial direction of the outer needle is converted to an axial direction of the port, and an inner peripheral wall of a wall surface on the port side or the outer needle side of the flow path is formed of the elastic member.

According to the configuration, it is possible to reduce a space in which blood may stagnate in the flow path between the outer needle and the port, and fluid can smoothly flow in the flow path.

Further, an indwelling needle according to an aspect of the present invention may be an indwelling needle including an inner needle that punctures a living body, an outer needle through which the inner needle penetrates, the outer needle being inserted into the living body together with the inner needle as the inner needle punctures the living body, a hub having a flow path communicating with an inside of the outer needle in a case where a base end of the outer needle is fixed and the inner needle is removed, and an elastic member that is arranged inside the hub and through which the inner needle penetrates, the elastic member closing a base end side of the hub in a case where the inner needle is removed. A port through which fluid flows to the flow path is provided on a side surface of the hub, the flow path has a conversion portion at least a part of which is formed of the elastic member and in which a flow direction of fluid flowing in from an axial direction of the outer needle is converted to an axial direction of the port, and in a case where the conversion portion is viewed from a direction perpendicular to both an axis of the outer needle and an axis of the port, both a wall surface on the outer needle side in the flow path and a wall surface on an opposite side to the outer needle have a curved shape projecting on the opposite side to the outer needle.

According to the configuration, it is possible to reduce a space in which blood may stagnate in the flow path on the opposing side to the conversion portion in which a flow direction of the flow path is changed, and fluid can smoothly flow in the flow path.

Further, in the indwelling needle in the above aspect, defining means for defining a relative position in a circumferential direction of the elastic member in the hub in a case of being viewed from an axial direction of the outer needle may be provided.

According to the configuration, in a case where the elastic member is provided with an opening through which fluid flows to the flow path, the position of the opening of the elastic member and the position of the opening of the port provided on a side surface of the hub can coincide with each other. That is, in a case where an external tube is connected to the port, the indwelling needle can be easily assembled so that fluid can circulate from the external tube to the flow path of the elastic member through the port.

### Advantageous Effects of Invention

According to the present invention, it is possible to provide a technique for suppressing stagnation of blood inside an indwelling needle.

### Brief Description of Drawings

FIG. 1 is a perspective view of an indwelling needle.
FIG. 2 is a trihedral figure of the indwelling needle.
FIG. 3 is a partially enlarged view of a cross section as viewed from arrow A-A in FIG. 2.
FIG. 4 is a perspective view of seal rubber.
FIG. 5 is four orthogonal views of the seal rubber.
FIG. 6A is a cross-sectional view of the seal rubber as viewed from arrow C-C in FIG. 5.
FIG. 6B is a cross-sectional view of the seal rubber as viewed from arrow D-D in FIG. 5.
FIG. 6C illustrates a relationship between a port axial direction, a direction perpendicular to the port axial direction, and each curvature.
FIG. 7 illustrates an example of a cross section perpendicular to a central axis direction in a state where the seal rubber is fitted into a hollow portion of a main body portion.
FIG. 8 is a perspective view of the indwelling needle.
FIG. 9 is a trihedral figure of the indwelling needle.
FIG. 10 is a partially enlarged view of a cross section as viewed from arrow A-A in FIG. 9.
FIG. 11 illustrates a cross section perpendicular to a central axis direction of a hub in the indwelling needle not provided with a rib or a groove. Description of Embodiment

Hereinafter, an embodiment of the present invention will be described. An embodiment below is an example of the embodiment of the present invention, and a technical scope of the present invention is not limited to an aspect below.

### <Outline of indwelling needle>

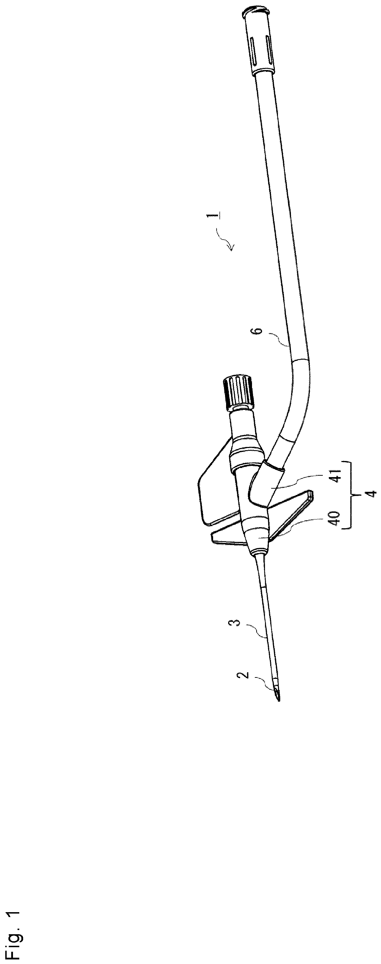

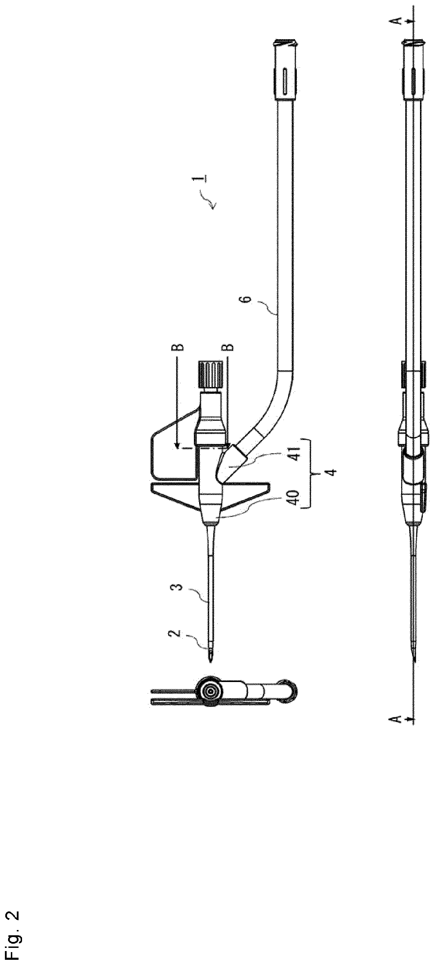

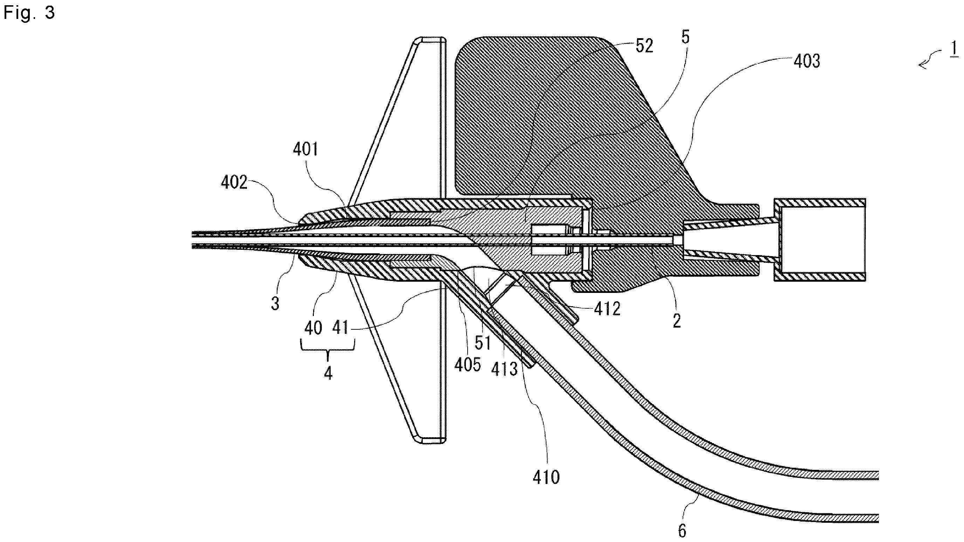

FIGS. 1 to 3 illustrate an example of an outline of an indwelling needle 1 according to the embodiment of the present invention. FIG. 1 is a perspective view of the indwelling needle 1. FIG. 2 is a trihedral figure of the indwelling needle 1. FIG. 3 is a partially enlarged view of a cross section as viewed from arrow A-A in FIG. 2.

First, an outline of the indwelling needle 1 will be described with reference to FIGS. 1 and 2. As illustrated in FIGS. 1 and 2, the indwelling needle 1 includes a hollow inner needle 2 and a cylindrical outer needle 3 provided to cover an outer surface of the inner needle 2. The inner needle 2 protrudes from an opening at a front end of the outer needle 3, and punctures a blood vessel of a patient to be punctured. Note that, in description below, an end portion (side/direction) of the indwelling needle 1 from which the inner needle 2 protrudes is referred to as a front end (side/direction), and an opposite end portion is referred to as a rear end (side/direction). Further, the direction in which the inner needle 2 protrudes is also referred to as a puncture direction.

An outer needle hub 4 is connected to a rear end of the outer needle 3. A hollow portion communicating with a hollow portion of the outer needle 3 is provided inside the outer needle hub 4. As illustrated in FIGS. 1 and 2, the outer needle hub 4 includes a main body portion 40 linearly extending in the puncture direction, and a branch portion (port) 41 branching in a Y shape in a direction opposite to the puncture direction from the main body portion 40. A hollow portion is provided inside both the main body portion 40 and the branch portion 41, and the hollow portions communicate with each other. Note that the outer needle hub 4 may be formed of a single member or a plurality of members. Further, a flexible soft tube may be arranged between a front end and a base end of the outer needle hub 4.

Further, an extension tube 6 through which blood supplied from an external device such as an artificial dialysis device passes is connected to a rear end of the branch portion 41 in the indwelling needle 1. Note that the extension tube 6 may not be provided, and a connector may be provided at a port end portion.

Next, details of the inside of the indwelling needle 1 will be described with reference to FIG. 3. As can be seen from FIG. 3, seal rubber 5 is arranged in the hollow portion of the main body portion 40 of the outer needle hub 4. The seal rubber 5 prevents leakage of blood from a rear end of the main body portion 40 and constitutes a flow path of blood as described later. Here, the seal rubber 5 is an example of the "elastic member" of the present invention, and may have any shape and configuration as long as it can prevent blood leakage from the rear end of the main body portion 40. Note that, in the present embodiment, the seal rubber 5 is compressed in a radial direction to suitably prevent leakage of blood.

Further, as illustrated in FIG. 3, the branch portion 41 is connected to the main body portion 40 at a connection portion 405. Then, the hollow portion of the branch portion 41 is provided with a connection hollow portion 413 near the connection portion 405 and an extension tube fitting portion 410 having an inner diameter larger than that of the connection hollow portion 413 and into which the extension tube 6 is inserted and fitted. Then, a region between the connection hollow portion 413 and the extension tube fitting portion 410 is provided with a tapered rear end hollow portion 412 whose inner diameter expands from an inner diameter equivalent to an inner diameter of the connection hollow portion 413 to an inner diameter equivalent to an inner diameter of the extension tube 6.

Further, an outer needle holding portion 401 that holds a rear end of the outer needle 3 is provided on the front end side of the hollow portion of the main body portion 40. Then, the front end of the outer needle holding portion 401 is provided with a front end opening 402 that opens in the puncture direction. The outer needle 3 is insert-molded in the outer needle holding portion 401 such that a front end of the outer needle 3 protrudes from the front end opening 402. Note that the outer needle holding portion 401 may be molded as a separate member, and a caulking pin for caulking the outer needle 3 and the outer needle hub 4 may be used. Further, the outer needle holding portion 401 may be an adhesive instead of a member. The hollow portion of the outer needle 3 is tapered in a front end direction from the rear end. The front end of the seal rubber 5 is externally inserted into a base end of the outer needle 3, and the front end of the seal rubber 5 extends further to the front end side than the port.

Further, the main body portion 40 of the outer needle hub 4 includes a rear end opening 403. The size of the rear end opening 403 is provided so that the seal rubber 5 can be inserted into the hollow portion of the outer needle hub 4.

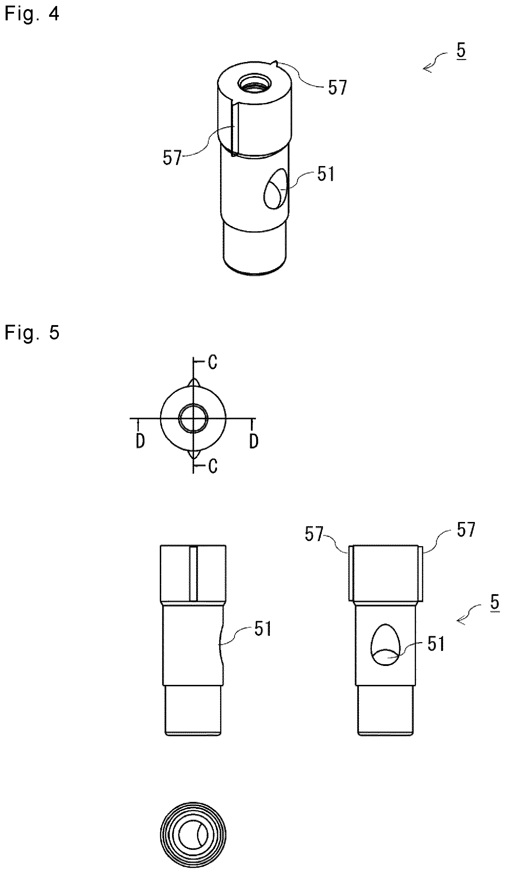

FIGS. 4 to 6 illustrate an example of an outline of the seal rubber 5.

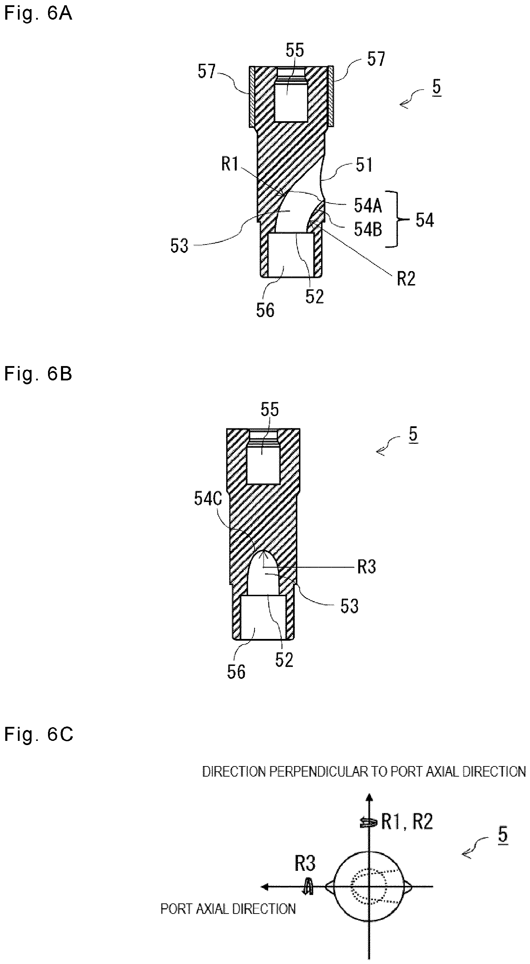

FIG. 4 is a perspective view of the seal rubber 5. Further, FIG. 5 is four orthogonal views of the seal rubber 5. Further, FIG. 6A is a cross-sectional view as viewed from arrow C-C in FIG. 5. Further, FIG. 6B is a cross-sectional view as viewed from arrow D-D in FIG. 5. Further, FIG. 6C illustrates a relationship between a port axial direction, a direction perpendicular to the port axial direction, and curvatures R1 to R3.

The seal rubber 5 is formed of, for example, an elastic material such as silicon, and has a substantially cylindrical outer shape as illustrated in FIGS. 4 and 5. Further, as can be seen from FIG. 6, the seal rubber 5 is provided with a flow path portion 53 for internally communicating a side surface of the seal rubber 5 and a front end surface. A rear end of the flow path portion 53 is opened on a side surface of the seal rubber 5, and forms an inlet 51 that allows blood to flow into the flow path portion 53 from the outside.

Here, a cross section of the inlet 51 perpendicular to an inflow direction of blood is circular. Then, an inner diameter of the cross section of the inlet 51 and an inner diameter of the connection hollow portion 413 are substantially the same.

In contrast, as illustrated in FIGS. 6A and 6B, an outer needle fitting portion 56 which is a columnar recess into which the rear end of the outer needle 3 is fitted is provided on the front end side of the seal rubber 5. In a vein side indwelling needle, an outlet 52 as an opening with respect to the outer needle fitting portion 56 is provided at a boundary with the outer needle fitting portion 56 in the flow path portion 53. That is, blood that has passed through the flow path portion 53 passes through the outlet 52 and flows into the hollow portion of the outer needle 3 fitted to the outer needle fitting portion 56. Then, an inner diameter of the outlet 52 is substantially the same as an inner diameter of an opening at the rear end of the outer needle 3 fitted to the outer needle fitting portion 56. Here, the flow path portion 53 converts a flowing direction of circulating blood between the inlet 51 and the outlet 52, and is an example of the "conversion portion" in the present invention. Here, in the present embodiment, the entire conversion portion is formed of the seal rubber 5. However, the configuration may be such that a part of the conversion portion is formed of the seal rubber 5. Note that, in an artery side indwelling needle, a flow direction of blood is opposite to that of the vein side indwelling needle, and the outlet 52 as an opening with respect to the outer needle fitting portion 56 serves as an entrance for flowing into the seal rubber 5 from the outer needle 3.

Further, as illustrated in FIG. 6A, the flow path portion 53 is formed by a side wall 54 having a curvature in a single direction without meandering between the inlet 51 and the outlet 52 in a cross section parallel to an inflow direction of blood flowing in from the inlet 51. Further, the flow path portion 53 has a circular cross section perpendicular to the inflow direction of blood and a diameter of the circle is substantially constant, and the flow path portion 53 is formed in a tunnel shape.

Here, as illustrated in FIG. 6A, when a cross section parallel to the inflow direction of blood flowing in from the inlet 51 is viewed, a side wall 54A and a side wall 54B, which are a part of the side wall 54 of the flow path portion 53, are changed at a predetermined curvature such that blood does not stagnate with respect to the central axis direction of the outer needle 3. That is, the side wall 54A and the side wall 54B have curved surfaces that change at a predetermined curvature (R1, R2) around a direction perpendicular to an opening direction of the inlet 51 (axial direction of the port) when viewed from the axial direction of the outer needle 3. Further, the side wall 54A and the side wall 54B project on the opposite side to the outer needle 3.

Further, as illustrated in FIG. 6B, when viewed from the axial direction of the outer needle 3, a side wall 54C of the flow path portion 53 is formed of a curved surface that changes at a predetermined curvature R3 around the opening direction of the inlet 51 (axial direction of the port).

Here, the predetermined curvatures R1, R2, and R3 are values at which blood does not stagnate. The value at which the blood does not stagnate may be calculated, for example, by simulation, or may be determined on the basis of a result of a measurement experiment.

Note that the same applies to the curvatures of the side wall 54A, the side wall 54B, and the side wall 54C when the side wall 54A, the side wall 54B, and the side wall 54C are viewed from the opening direction of the inlet 51 (axial direction of the port). That is, as illustrated in FIG. 6A, the side wall 54A and the side wall 54B, which are a part of the side wall 54 of the flow path portion 53, have curved surfaces that change at a predetermined curvature (R1, R2) around the direction perpendicular to the axial direction of the outer needle 3 even when viewed from the opening direction of the inlet 51 (axial direction of the port).

Further, also when viewed from the opening direction of the inlet 51 (the axial direction of the port), the side wall 54C of the flow path portion 53 is formed of a curved surface that changes at the predetermined curvature R3 around the axial direction of the outer needle 3 (in FIG. 6B, the side wall 54C of the flow path portion 53 has a curved surface at the curvature R3 also in a front-back direction of the drawing).

Further, in the present embodiment, as illustrated in FIG. 6A, the side wall 54B on the inlet 51 side (port side) and the outer needle 3 side (outer needle side) of the flow path portion 53 is formed of the seal rubber 5 (elastic member).

Further, as illustrated in FIG. 3, the seal rubber 5 is configured such that the inner needle 2 penetrates, and a recess 55 into which the inner needle 2 is inserted when the inner needle 2 penetrates the seal rubber 5 is provided in the inside near a rear end of the seal rubber 5.

On an outer surface near the rear end of the seal rubber 5, ribs 57 protruding in a direction perpendicular to the central axis direction and formed along the central axis direction are provided at two places at intervals of 180 degrees. Here, a groove 404 is provided along the central axis direction on an inner wall of the hollow portion of the main body portion 40 of the outer needle hub 4, and a posture of the seal rubber 5 around the central axis in the main body portion 40 can be determined as the rib 57 of the seal rubber 5 is fitted to the groove 404.

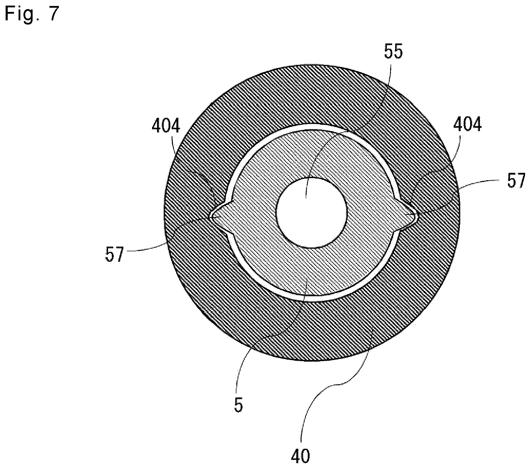

FIG. 7 illustrates an example of a cross section perpendicular to the central axis direction (cross-sectional view as viewed in arrow B-B in FIG. 2) in a state where the seal rubber 5 is inserted into the hollow portion of the main body portion 40. Here, the position where the rib 57 and the groove 404 are provided is determined such that the position of the inlet 51 of the seal rubber 5 coincides with the position of an opening of the connection hollow portion 413 when the seal rubber 5 is inserted into the hollow portion of the main body portion 40. Here, the rib 57 and the groove 404 are an example of the "defining means" of the present invention.

Further, as described above, an inner diameter of a portion opening in a rear end direction of the rear end hollow portion 412 is provided to be substantially the same as an inner diameter of the extension tube 6. With such a structure, the connection hollow portion 413 of the branch portion 41 and a hollow portion of the extension tube 6 smoothly communicate with each other.

### (Assembly procedure of indwelling needle 1)

Next, an example of an outline of an assembly procedure of the indwelling needle 1 will be described. First, the seal rubber 5 is inserted into a hollow portion of the main body portion 40 from the rear end opening 403 provided at the rear end of the outer needle hub 4. When the seal rubber 5 is inserted, the rib 57 provided on an outer surface of the seal rubber 5 and the groove 404 provided on an inner surface of the outer needle hub 4 are inserted so as to be fitted. Then, the outer needle fitting portion 56 of the seal rubber 5 inserted into the hollow portion of the main body portion 40 is fitted to the rear end of the outer needle 3 insert-molded in the outer needle holding portion 401. Note that the outer needle 3 and the seal rubber 5 may be assembled to the outer needle hub 4 by inserting the outer needle 3 molded separately into the outer needle hub 4 in a state of being assembled to the outer needle fitting portion 56 of the seal rubber 5.

Here, an inner diameter of the outlet 52 of the seal rubber 5 is provided to be substantially the same as an inner diameter of the outer needle 3 fitted to the outer needle fitting portion 56. Therefore, the side wall 54 forming the flow path portion 53 and the side wall forming the hollow portion of the outer needle 3 are smoothly continuous via the outlet 52. Further, when the outer needle fitting portion 56 is fitted to the rear end of the outer needle 3, a front end of the seal rubber 5 enters a gap of an outer shape of the outer needle 3, the outer needle hub 4, and an inner wall. In this manner, a space between the outer needle 3 and the outer needle hub 4 is sealed. Here, the seal rubber 5 entering the gap between the outer needle 3 and the outer needle hub 4 is an example of the "seal portion" of the present invention.

Further, in a case where the insertion of the seal rubber 5 into the hollow portion of the main body portion 40 is completed, the position of the inlet 51 coincides with the opening of the connection hollow portion 413 inside the branch portion 41. Therefore, the side wall 54 and a side wall of the connection hollow portion 413 are smoothly continuous via the inlet 51.

Further, the extension tube 6 is inserted into the extension tube fitting portion 410. Therefore, the hollow portion of the extension tube 6 and the connection hollow portion 413 of the branch portion 41 communicate with each other via the rear end hollow portion 412. Here, an inner diameter of a rear end of the rear end hollow portion 412 is substantially the same as an inner diameter of the extension tube 6. Therefore, a side wall of the rear end hollow portion 412 and a side wall of the hollow portion of the extension tube 6 are smoothly continuous.

Next, the inner needle 2 is inserted into the recess 55 of the seal rubber 5 at a predetermined position through the rear end opening 403 of the outer needle hub 4. In this manner, a front end of the inner needle 2 protrudes from a predetermined position of the side wall 54 forming the flow path portion 53 to the flow path portion 53. Furthermore, the inner needle 2 keeps passing through the inside of the flow path portion 53, enters the hollow portion of the outer needle 3, and protrudes from an opening at a front end of the outer needle 3. In a state where the inner needle 2 protrudes from the opening at the front end of the outer needle 3 as described above, the inner needle 2 can puncture a blood vessel of a patient.

### (Usage procedure of indwelling needle 1)

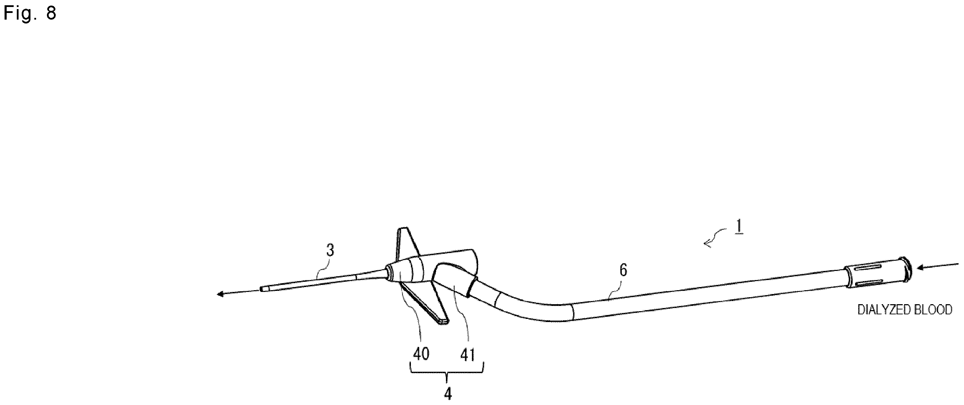

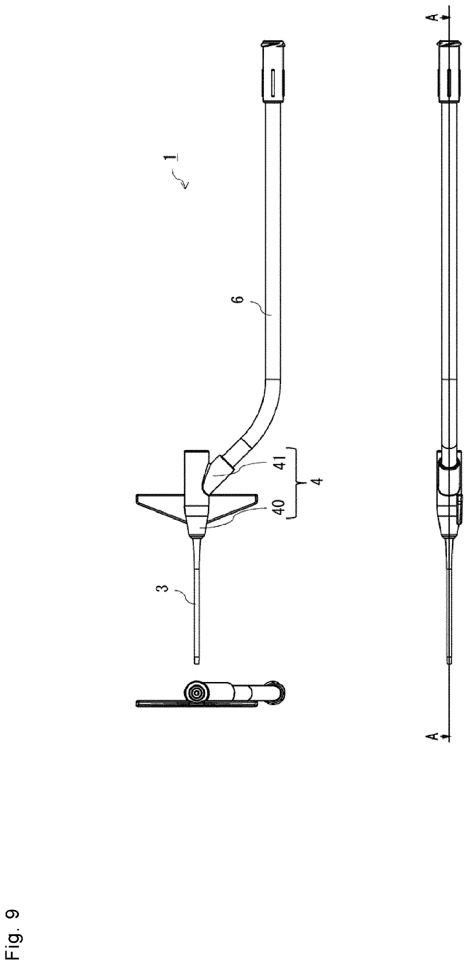

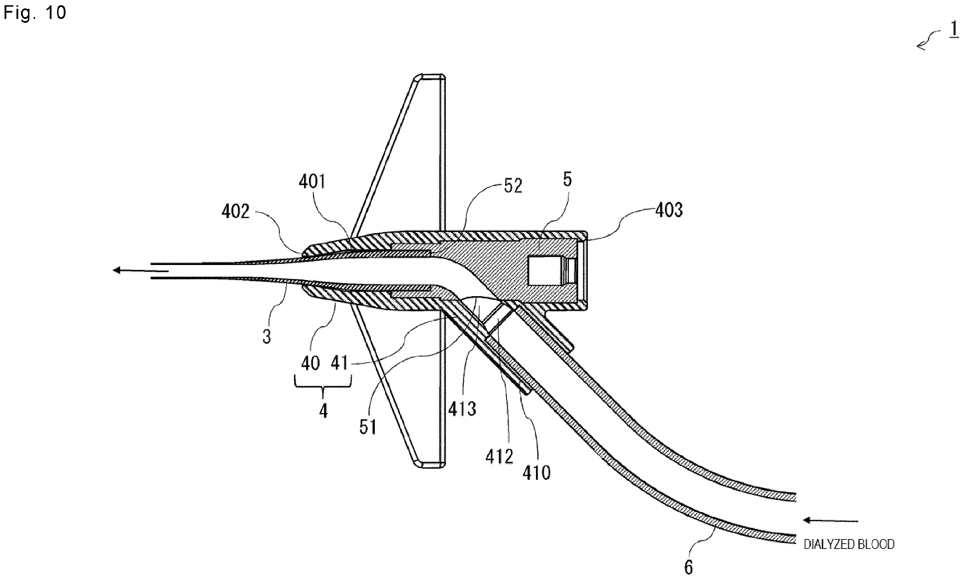

Next, an example of an outline of a usage procedure of the indwelling needle 1 will be described. After puncturing a blood vessel of a patient, the inner needle 2 is removed in a direction opposite to the puncture direction. FIGS. 8 to 10 illustrate an example of the indwelling needle 1 in a state where the inner needle 2 is removed. FIG. 8 is a perspective view of the indwelling needle 1. FIG. 9 is a trihedral figure of the indwelling needle 1. FIG. 10 is a partially enlarged view of a cross section as viewed from arrow A-A in FIG. 9.

Dialysis treatment is performed by allowing blood to flow from a blood vessel into an extracorporeal circuit (blood removal) and returning blood that has passed through a blood purifier in the extracorporeal circuit to a blood vessel (blood return), and for the treatment, an artery side indwelling needle to be punctured into the artery side and a vein side indwelling needle to be punctured into the vein side are used. The present invention is used for at least one or both of the artery side indwelling needle and the vein side indwelling needle.

When the indwelling needle 1 punctures a blood vessel and the inner needle 2 is removed, the outer needle 3 is left indwelling in the blood vessel of a patient. Further, a through hole of the seal rubber 5 through which the inner needle 2 has been inserted is closed by elasticity of the seal rubber 5. Therefore, blood in a blood vessel of a patient is prevented from flowing backward through the outer needle 3 and leaking to the outside from the through hole of the seal rubber 5 through which the inner needle 2 has been inserted.

After the inner needle 2 is removed, an extracorporeal circuit is connected to a connector of the extension tube 6, and a flow path is formed by an artery side blood vessel, a blood purifier, and a vein side blood vessel. In the indwelling needle on the artery side, blood flows from the outer needle 3 toward the branch portion 41, and at that time, the blood smoothly flows from the outer needle 3 to the branch portion 41 via the flow path portion 53.

Then, dialyzed blood that has passed through the blood purifier flows into the hollow portion of the outer needle 3 from the branch portion 41. In the indwelling needle on the vein side, blood flows from the branch portion 41 toward the outer needle 3, and at that time, blood smoothly flows from the branch portion 41 to the outer needle 3 via the flow path portion 53. Since the hollow portion of the outer needle 3 is tapered from a terminal in a front end direction, a flow rate of the dialyzed blood increases. Then, the dialyzed blood is supplied into a blood vessel of a patient through an opening at the front end of the outer needle 3 left indwelling in a blood vessel of the patient.

### [Operation and effect]

According to the indwelling needle 1 as described above, since blood smoothly flows from an inlet toward an outlet by the flow path portion 53, stagnation hardly occurs. The side wall 54A and the side wall 54B of the flow path portion 53 both project to the side opposite to the outer needle 3. That is, since the side walls 54A and 54B forming the flow path portion 53 do not have a complicated shape such as meandering, stagnation of blood in the flow path portion 53 is suppressed. Further, in the flow path portion 53 forming a flow path for converting a flow path direction, a cross section perpendicular to an inflow direction of blood has a circular shape, and an inner diameter of the circle is substantially constant. Therefore, stagnation of blood in the flow path portion 53 is suppressed. Since stagnation of blood is suppressed, formation of a thrombus in the flow path portion 53 is suppressed. Note that, in addition to an elliptical shape, an oval shape, and an egg shape, a flow path having a substantially circular shape and a slight variation in radius can be used. Also in this case, since the flow path cross section is circular, blood can be formed so as to be guided to the center of the flow path cross section, and generation of a stagnation portion in a part of the flow path can be prevented. Further, the cross section of the flow path portion 53 can also be formed of seal rubber and an outer needle hub.

Further, according to the indwelling needle 1 as described above, as illustrated in FIG. 6A, when a cross section parallel to the inflow direction of blood flowing in from the inlet 51 is viewed, the side wall 54A and the side wall 54B, which are a part of the side wall 54 of the flow path portion 53, are changed at a predetermined curvature such that blood does not stagnate with respect to the central axis direction of the outer needle 3. That is, the side wall 54A and the side wall 54B have curved surfaces that change at a predetermined curvature (R1, R2) around a direction perpendicular to an opening direction of the inlet 51 (axial direction of the port) when viewed from the axial direction of the outer needle 3.

Further, as illustrated in FIG. 6B, when viewed from the axial direction of the outer needle 3, the side wall 54C of the flow path portion 53 is formed of a curved surface that changes at the predetermined curvature R3 around the opening direction of the inlet 51 (axial direction of the port).

Here, the predetermined curvatures R1, R2, and R3 are values at which blood does not stagnate. Therefore, according to the indwelling needle 1 as described above, stagnation of blood in the flow path portion 53 is suppressed.

In addition, according to the indwelling needle 1 as described above, the flow path portion 53 of the seal rubber 5 is formed in a tunnel shape, and a smooth flow path without a step can be realized. For this reason, it is possible to prevent stagnation of blood, and it is also possible to prevent formation of a thrombus due to stagnation. Further, the side wall 54 of the flow path portion 53 is not formed of a surface of a member other than the seal rubber 5. Therefore, stagnation of blood at a joint with a surface of another member on the side wall 54 is suppressed.

Further, according to the indwelling needle 1 as described above, an inner diameter of the inlet 51 is substantially the same as an inner diameter of the connection hollow portion 413 of the connection portion 405 of the outer needle hub 4. Further, according to the indwelling needle 1 described above, the position where the rib 57 of the seal rubber 5 and the groove 404 of the outer needle hub 4 are provided is a position where the inlet 51 coincides with the connection hollow portion 413 when the seal rubber 5 is inserted into the hollow portion of the main body portion 40 of the outer needle hub 4. Further, a cross-sectional shape of an opening portion of the connection hollow portion 413 with respect to the flow path portion 53 is the same as an opening shape of the inlet 51 with respect to the connection hollow portion 413.

Therefore, the side wall 54 forming the flow path portion 53 and a side wall inside the connection hollow portion 413 are smoothly continuous. In this manner, stagnation of blood at a connection portion between the flow path portion 53 and the connection hollow portion 413 is suppressed. Further, according to the indwelling needle 1 described above, it is possible to easily assemble the indwelling needle 1 so as to allow blood to flow from the extension tube 6 to the flow path portion 53 via the connection hollow portion 413.

Further, according to the indwelling needle 1 as described above, the side wall 54 forming the flow path portion 53 of the seal rubber 5 is smoothly continuous with a side wall forming the hollow portion of the outer needle 3 on the outlet 52 side. Therefore, at a joint between the side wall 54 of the seal rubber 5 and the side wall forming the hollow portion of the outer needle 3, stagnation of blood supplied to a blood vessel of a patient is suppressed.

Further, according to the indwelling needle 1 as described above, a space between the outer needle 3 and an inner wall of the outer needle hub 4 is sealed by the seal rubber 5. Therefore, blood flowing toward the inside of the outer needle 3 through the flow path portion 53 of the seal rubber 5 is prevented from flowing into and stagnating in a gap between the outer needle 3 and the inner wall of the outer needle hub 4. Further, unlike the above embodiment, even in a case where blood is collected from a blood vessel of a patient, it is possible to prevent blood passing through the inside of the outer needle 3 and flowing toward the flow path portion 53 of the seal rubber 5 from flowing into and stagnating in the gap between the outer needle 3 and the inner wall of the outer needle hub 4.

Further, according to the indwelling needle 1 as described above, the side wall 54B on the inlet 51 side (port side) and the outer needle 3 side (outer needle side) of the flow path portion 53 is formed of the seal rubber 5 (elastic member). Therefore, a joint between the flow path portion 53 and the port and a joint between the flow path portion 53 and the outer needle 3 can be made smoothly continuous by the elastic member. Therefore, stagnation of blood is suppressed at the joint between the flow path portion 53 and the port or the joint between the flow path portion 53 and the outer needle 3.

Further, according to the indwelling needle 1 as described above, a length from the outer needle 3 to a base end of the outer needle hub 4 can be shortened. Therefore, even in a case where a safety mechanism for preventing a puncture accident of the inner needle 2 after removal of the inner needle 2 is provided between the outer needle hub 4 and the inner needle hub, it is possible to prevent an increase in the overall size. Note that the safety mechanism is not limited to a configuration provided between the outer needle hub 4 and the inner needle hub, and another safety mechanism used in an indwelling needle having the inner needle 2 and the outer needle 3 may be employed.

Further, a shape or a position of the rib 57 and the groove 404 are not limited to those in the above embodiment, and the groove may be provided on an outer surface of the seal rubber and the rib may be provided on an inner surface of the outer needle hub. In a state where the seal rubber 5 is inserted into the hollow portion of the main body portion 40, the inlet 51 of the seal rubber 5 preferably has a shape or a position that coincide with an opening of the connection hollow portion 413 inside the connection portion 405 from the main body portion 40 of the outer needle hub 4. However, the configuration is not limited to this. Further, the configuration may be such that relative positions of the outer needle hub and the seal rubber in the circumferential direction are defined without providing the rib 57 and the groove 404.

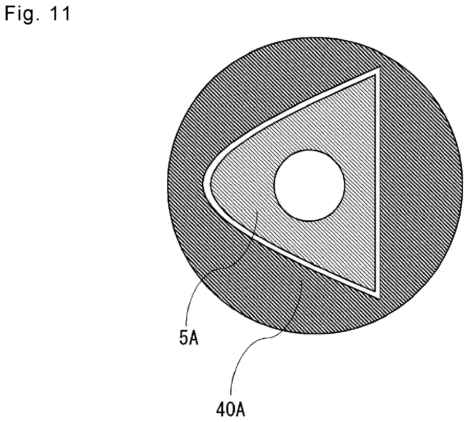

FIG. 11 illustrates a cross section perpendicular to a central axis direction of an outer needle hub 4A in an indwelling needle 1A in which the rib 57 and the groove 404 are not provided. In the case of the indwelling needle 1A illustrated in FIG. 11, the hollow portion of a main body portion 40A of the outer needle hub 4A and seal rubber 5A are not rotationally symmetric about the central axis. That is, a relative position in a circumferential direction of the seal rubber 5A in the outer needle hub 4A is defined. Therefore, when the seal rubber 5A is inserted into the hollow portion of the main body portion 40A as illustrated in FIG. 11, the main body portion 40A and the seal rubber 5A only need to be manufactured such that an inlet 51A of the seal rubber 5A of the indwelling needle 1A coincides with an opening of a connection hollow portion 413A inside a connection portion 405Afrom the main body portion 40A.

Further, the seal rubber 5 may be surrounded by a hard member relatively harder than the seal rubber 5, a rib or a groove may be provided on an outer surface of the hard member, and an engagement mechanism that engages with the rib or the groove provided on the outer surface of the hard member may be provided on an inner surface of the outer needle hub 4. Then, in a state where the rib or the groove of the hard member and the engagement mechanism on an inner surface of the outer needle hub 4 are engaged and the seal rubber 5 is inserted into the hollow portion of the main body portion 40, the inlet 51 of the seal rubber 5 may coincide with an opening of the connection hollow portion 413 inside the connection portion 405 from the main body portion 40 of the outer needle hub 4.

Further, the above embodiment may further include a mechanism that facilitates flashback. For example, if a side hole is provided in the inner needle 2 and the inner needle 2 and the extension tube 6 communicate with each other, the user can confirm that the indwelling needle 1 punctures a blood vessel by movement of blood between the inner needle 2 and the outer needle hub 4 or movement of blood in the extension tube 6. Further, as a luer connector is provided at an end portion of the extension tube 6, a valve can also be provided in the luer connector. The luer connector may be provided with a cap, or the extension tube 6 may be provided with a clamp. A publicly-known ventilation mechanism can also be provided so that blood can be removed up to an end portion of the extension tube 6.

The embodiment and variation disclosed above can be combined.

### Reference Signs List

- 1, 1A: indwelling needle
- 2: inner needle
- 3: outer needle
- 4, 4A: outer needle hub
- 5, 5A: seal rubber
- 6: extension tube
- 40, 40A: main body portion
- 41: branch portion
- 51, 51A: inlet
- 52: outlet
- 53: flow path portion
- 54, 54A, 54B, 54C: side wall
- 55: recess
- 56: outer needle fitting portion
- 57: rib
- 401: outer needle holding portion
- 402: front end opening
- 403: rear end opening
- 404: groove
- 405, 405A: connecting portion
- 410: extension tube fitting portion
- 412: rear end hollow portion
- 413, 413A: connection hollow portion

## Claims

1. An indwelling needle comprising:
an inner needle that punctures a living body;
an outer needle through which the inner needle penetrates, the outer needle being inserted into the living body together with the inner needle as the inner needle punctures the living body;
a hub having a flow path communicating with an inside of the outer needle in a case where a base end of the outer needle is fixed and the inner needle is removed; and
an elastic member that is arranged inside the hub and through which the inner needle penetrates, the elastic member closing a base end side of the hub in a case where the inner needle is removed, wherein
a port through which fluid flows to the flow path is provided on a side surface of the hub,
the flow path includes a conversion portion in which a flow direction of fluid flowing in from an axial direction of the outer needle is converted to an axial direction of the port or a flow direction of fluid flowing in from the axial direction of the port is converted to the axial direction of the outer needle, and
at least a part of the flow path in the conversion portion is formed of the elastic member, and has a cross section of a circular shape.

2. An indwelling needle comprising:
an inner needle that punctures a living body;
an outer needle through which the inner needle penetrates, the outer needle being inserted into the living body together with the inner needle as the inner needle punctures the living body;
a hub having a flow path communicating with an inside of the outer needle in a case where a base end of the outer needle is fixed and the inner needle is removed; and
an elastic member that is arranged inside the hub and through which the inner needle penetrates, the elastic member closing a base end side of the hub in a case where the inner needle is removed, wherein
a port through which fluid flows to the flow path is provided on a side surface of the hub,
the flow path has a conversion portion at least a part of which is formed of the elastic member and in which a flow direction of fluid flowing in from an axial direction of the outer needle is converted to an axial direction of the port,
at least a part of a connection portion between the flow path and the port is formed of the elastic member, and
a cross-sectional shape of an opening portion of the port with respect to the flow path in the connection portion is the same as a cross-sectional shape of an opening portion of the flow path with respect to the port.

3. An indwelling needle comprising:
an inner needle that punctures a living body;
an outer needle through which the inner needle penetrates, the outer needle being inserted into the living body together with the inner needle as the inner needle punctures the living body;
a hub having a flow path communicating with an inside of the outer needle in a case where a base end of the outer needle is fixed and the inner needle is removed; and
an elastic member that is arranged inside the hub and through which the inner needle penetrates, the elastic member closing a base end side of the hub in a case where the inner needle is removed and connecting with the base end of the outer needle at a front end, wherein
a port through which fluid flows to the flow path is provided on a side surface of the hub,
the flow path has a conversion portion at least a part of which is formed of the elastic member and in which a flow direction of fluid flowing in from an axial direction of the outer needle is converted to an axial direction of the port, and
the front end of the elastic member extends further on a front end side than the port.

4. An indwelling needle comprising:
an inner needle that punctures a living body;
an outer needle through which the inner needle penetrates, the outer needle being inserted into the living body together with the inner needle as the inner needle punctures the living body;
a hub having a flow path communicating with an inside of the outer needle in a case where a base end of the outer needle is fixed and the inner needle is removed; and
an elastic member that is arranged inside the hub and through which the inner needle penetrates, the elastic member closing a base end side of the hub in a case where the inner needle is removed, wherein
a port through which fluid flows to the flow path is provided on a side surface of the hub, and
at least a part of the flow path is a tunnel-shaped flow path formed in the elastic member.

5. An indwelling needle comprising:
an inner needle that punctures a living body;
an outer needle through which the inner needle penetrates, the outer needle being inserted into the living body together with the inner needle as the inner needle punctures the living body;
a hub having a flow path communicating with an inside of the outer needle in a case where a base end of the outer needle is fixed and the inner needle is removed; and
an elastic member that is arranged inside the hub and through which the inner needle penetrates, the elastic member closing a base end side of the hub in a case where the inner needle is removed, wherein
a port through which fluid flows to the flow path is provided on a side surface of the hub,
the flow path has a conversion portion at least a part of which is formed of the elastic member and in which a flow direction of fluid flowing in from an axial direction of the outer needle is converted to an axial direction of the port, and
an inner peripheral wall of a wall surface on the port side or the outer needle side of the flow path is formed of the elastic member.

6. An indwelling needle comprising:
an inner needle that punctures a living body;
an outer needle through which the inner needle penetrates, the outer needle being inserted into the living body together with the inner needle as the inner needle punctures the living body;
a hub having a flow path communicating with an inside of the outer needle in a case where a base end of the outer needle is fixed and the inner needle is removed; and
an elastic member that is arranged inside the hub and through which the inner needle penetrates, the elastic member closing a base end side of the hub in a case where the inner needle is removed, wherein
a port through which fluid flows to the flow path is provided on a side surface of the hub,
the flow path has a conversion portion at least a part of which is formed of the elastic member and in which a flow direction of fluid flowing in from an axial direction of the outer needle is converted to an axial direction of the port, and
in a case where the conversion portion is viewed from a direction perpendicular to both an axis of the outer needle and an axis of the port, both a wall surface on the outer needle side in the flow path and a wall surface on an opposite side to the outer needle have a curved shape projecting on the opposite side to the outer needle.

7. The indwelling needle according to any one of claims 1 to 6, wherein defining means for defining a relative position in a circumferential direction of the elastic member in the hub in a case of being viewed from an axial direction of the outer needle is provided.
